(19) Europäisches Patentamt

European Patent Office

Office européen des brevets

(11) **EP 0 582 931 B1**

(12) **EUROPÄISCHE PATENTSCHRIFT**

(45) Veröffentlichungstag und Bekanntmachung des Hinweises auf die Patenterteilung:
**03.09.1997 Patentblatt 1997/36**

(51) Int. Cl.$^6$: **C07C 68/06**, C07C 69/96

(21) Anmeldenummer: **93112354.1**

(22) Anmeldetag: **02.08.1993**

(54) **Verfahren zur kontinuierlichen Herstellung von Diarylcarbonaten aus Dialkylcarbonaten**

Process for the continuous preparation of diarylcarbonates from dialkylcarbonates

Procédé pour la préparation en continue de diarylcarbonates à partir de dialkylcarbonates

(84) Benannte Vertragsstaaten:
**BE DE ES FR GB IT NL**

(30) Priorität: **13.08.1992 DE 4226755**

(43) Veröffentlichungstag der Anmeldung:
**16.02.1994 Patentblatt 1994/07**

(73) Patentinhaber: **BAYER AG**
**51368 Leverkusen (DE)**

(72) Erfinder:
 • **Schön, Norbert, Dr.**
 **D-47800 Krefeld (DE)**
 • **Langer, Reinhard, Dr.**
 **D-47800 Krefeld (DE)**
 • **Buysch, Hans-Josef, Dr.**
 **D-47809 Krefeld (DE)**
 • **Wagner, Paul, Dr.**
 **D-40597 Düsseldorf (DE)**

(56) Entgegenhaltungen:
 **EP-A- 0 461 274**          **DE-A- 2 503 195**

 • **PATENT ABSTRACTS OF JAPAN vol. 11 no. 163 (C-424) [2610] ,26.Mai 1987 & JP-A-61 291545 (DAICEL CHEM IND LTD)**
 • **PATENT ABSTRACTS OF JAPAN vol. 11 no. 163 (C-424) [2610] ,26.Mai 1987 & JP-A-61 291545**

**Beschreibung**

Die Erfindung betrifft ein kontinuierlich arbeitendes Verfahren zur Herstellung von Diarylcarbonaten aus Dialkylcarbonaten und Phenolen unter Verwendung von üblichen Umesterungskatalysatoren, das dadurch gekennzeichnet ist, daß die Ausgangsstoffe in einer speziellen, stofflich und energetisch gekoppelten Kombination von Kolonnen umgesetzt werden

Die Herstellung von aromatischen und aliphatisch-aromatischen Kohlensäureestern (Carbonaten) durch Umesterung, ausgehend von aliphatischen Kohlensäureestern und Phenolen, ist im Prinzip bekannt. Dabei handelt es sich um eine Gleichgewichtsreaktion, wobei die Lage des Gleichgewichts fast vollständig in Richtung der aliphatisch substituierten Carbonate verschoben ist. Daher ist es verhältnismäßig leicht, aus aromatischen Carbonaten und Alkoholen aliphatische Carbonate herzustellen. Um die Reaktion jedoch im umgekehrten Sinne in Richtung aromatischer Carbonate durchzuführen, ist es notwendig, das sehr ungünstig liegende Gleichgewicht effektiv zu verschieben, wobei nicht nur sehr aktive Katalysatoren, sondern auch eine günstige Verfahrensweise zur Anwendung gelangen müssen.

Zur Umesterung von aliphatischen Kohlensäureestern mit Phenolen sind eine Vielzahl von effektiven Katalysatoren, wie beispielsweise Alkalihydroxide, Lewissäure-Katalysatoren aus der Gruppe der Metallhalogenide (DE-OS 25 28 412 und DE-OS 25 52 907), Organozinnverbindungen (EP-879, EP-880, DE-OS 34 45 552, EP-338 760), Bleiverbindungen (JP-57/176 932), Lewissäure/Protonensäure-Katalysatoren (DE-OS 34 45 553), empfohlen worden. In den bekannten Verfahren wird die Umesterung in einem chargenweise betriebenen Reaktor drucklos oder unter Druck, gegebenenfalls mit einer zusätzlichen Trennkolonne, durchgeführt. Dabei werden auch mit den aktivsten Katalysatoren Reaktionszeiten von vielen Stunden bis zum Erreichen auch nur mittlerer Umsätze von ungefähr 50 % an Phenol benötigt. So werden bei der chargenweise betriebenen Umesterung von Phenol mit Diethylcarbonat bei 180°C unter Verwendung verschiedener Organozinnverbindungen, wie sie in DE-OS 34 45 552 beschrieben werden, Ausbeuten an Diphenylcarbonat in einer Größenordnung von mehr als 20 % erst nach ca. 24-stündiger Reaktionszeit erreicht; bei der chargenweise betriebenen Umesterung von Phenol und Dimethylcarbonat mit Hilfe von Organozinnkatalysatoren, wie sie in EP-879 beschrieben sind, beträgt der Phenolumsatz nach 30 Stunden 34 % des theoretischen Wertes.

Das bedeutet, daß aufgrund der ungünstigen thermodynamischen Voraussetzungen die beschriebenen Umesterungsreaktionen in Kesseln oder Druckautoklaven auch bei Verwendung sehr aktiver Katalysatorsysteme im Sinne eines technischen Prozesses nur sehr unvorteilhaft durchführbar sind, da sich sehr schlechte Raum-Zeit-Ausbeuten und hohe Verweilzeiten bei hohen Reaktionstemperaturen ergeben, wobei wegen der unvollständigen Umesterung zusätzlich ein hoher Destillationsaufwand aufgebracht werden muß, der weitere Energie erforderlich macht.

Solche Verfahrensweisen sind auch deshalb besonders unvorteilhaft, da selbst mit sehr selektiven Umesterungskatalysatoren bei den hohen Temperaturen und langen Verweilzeiten von vielen Stunden ein merklicher Anteil an Nebenreaktionen auftritt, beispielsweise die Etherbildung und die Abspaltung von Kohlendioxid.

Es wurde daher versucht, das Reaktionsgleichgewicht durch Adsorption des bei der Umesterung entstehenden Alkohols an Molekularsieben möglichst schnell in Richtung der gewünschten Produkte zu verschieben (DE-OS 33 08 921). Aus der Beschreibung dieser Reaktion zeigt sich, daß zur Adsorption des Reaktionsalkohols eine große Menge an Molekularsieb benötigt wird, die die Menge an freiwerdendem Alkohol weit überschreitet. Weiterhin müssen die eingesetzten Molekularsiebe schon nach kurzer Zeit regeneriert werden, und die Umwandlungsrate zu den Alkylarylcarbonat-Zwischenprodukten ist relativ gering. Auch dieses Verfahren erscheint deshalb als technisch nicht vorteilhaft anwendbar.

Es ist bekannt, Gleichgewichtsreaktionen, inbesondere Veresterungen und Umesterungen, in Kolonnnen durchzuführen und sie auf diese Weise vorteilhalt in Richtung Produktbildung zu verschieben (z.B. U.Block, Chem.-Ing.-Techn. 49, 151 (1977); DE-OS 3 809 417; B.Schleper, B.Gutsche, J.Wnuck und L.Jeromin, Chem.-Ing.-Techn. 62, 226 (1990); Ullmanns Encyclopädie der techn. Chemie, 4. Aufl., Bd. 3, S. 375ff. 1973; ibid, 5.Aufl., Bd. B4, S. 321, 1992).

In EP 0 461 274 (WO 91/09832) wird ein kontinuierlicher Umesterungsprozeß zur Herstellung von aromatischen Carbonaten in einer oder in mehreren hintereinander geschalteten mehrstufigen Kolonnen beschrieben, wobei Dialkylcarbonate oder Alkylarylcarbonate mit Phenolen umgesetzt werden und am Kopf der Kolonnen die leichtflüchtigen Produkte, nämlich Reaktionsalkohole und Dialkylcarbonate, und am Fuß der Kolonnen die schwersiedenden Produkte, nämlich Arylcarbonate, entommen werden.

Es wird also hier ein schon bekanntes Verfahrensprinzip, die Durchführung von Umesterungsreaktionen in Kolonnen, auf ein spezielles Problem, nämlich auf die Umesterung von Alkylcarbonaten zu Arylcarbonaten, angewendet. Besondere Maßnahmen technischen Handelns, die eine vorteilhaftere Durchführung der Umesterung unter Anpassung der Apparate und Vorgehensweisen an die oben angegebenen speziellen Probleme dieser schwierigen Umesterung erlauben, sind jedoch nicht angegeben. So wird beispielsweise die Art der Dosierung der beiden Edukte, Alkylcarbonat und aromatische Hydroxyverbindung, nicht klar definiert und auch keine vorteilhafte Fahrweise hervorgehoben. In einer Fahrweise nach Abb.1 der EP 0 461 274 werden beispielsweise Gemische dieser beiden Edukte in den oberen Teil der Kolonne eingespeist, die leichtsiedenden Reaktionsprodukte, nämlich Alkohole und nicht umgesetztes Dialkylcarbonat, am Kopf der Kolonne und die schwersiedenden Reaktionsprodukte Alkylaryl- und Diarylcarbonate zusammen mit nicht umgesetzten Dialkylcarbonaten und aromatischen Hydroxyverbindungen am Fuß der Kolonne entnommen. In der Fahrweise nach Abb.2 der EP 0 461 274 werden Gemische von Alkylcarbonaten und aromatischen Hydroxyverbindungen an zwei verschiedenen Punkten der Kolonne, nämlich im oberen und im unteren Drittel der Kolonne zugespeist und Edukt/Produkt-Gemische wie in der Fahrweise nach Abb.1 der EP 0 461 274 entnommen. Zwischen einer Führung der Edukte im Gleichstrom und im Gegenstrom wird weder in der Offenbarung noch in den Beispielen klar unterschieden, obwohl sie von großem Einfluß auf das Verfahrensresultat sein können.

Ferner wird auf den Einfluß von Temperatur, Druck, Katalysatorkonzentration und Flüssigverweilzeit nicht eingegangen, sondern es werden nur sehr weite Bereichsangaben, auch in den eingeschränkten Ansprüchen gemacht, beispielsweise werden Temperaturbereiche von 100 bis 280°C, Druckbereiche von 0,1 bis 200 bar, Katalysatorkonzentrationen von 0,001 bis 50 Gew.-% und Flüssigverweilzeiten von 0,05 bis 2 h angegeben.

Auf unterschiedliche und jeweils zu bevorzugende Verfahrensweisen für die einzelnen bei der Umsetzung von Dialkylcarbonaten zu Diarylcarbonaten auftretenden Reaktionen, beispielsweise die erste Umesterungsstufe von Dialkylcarbonaten mit aromatischen Hydroxyverbindungen zu Alkylarylcarbonaten nach Gleichung 1, die zweite Umesterungsstufe zu Diarylcarbonaten nach Gleichung 2 und die Disproportionierung gemäß Gleichung 3 wird in der Offenbarung nicht eingegangen.

$$\text{Alk-O-CO-O-Alk} + \text{Ar-OH} \rightarrow \text{Alk-O-CO-O-Ar} + \text{Alk-OH} \qquad \text{(Gl. 1)}$$

$$\text{Alk-O-CO-O-Ar} + \text{ArOH} \rightarrow \text{Ar-O-CO-O-Ar} + \text{Alk-OH} \qquad \text{(Gl. 2)}$$

$$2\ \text{Alk-O-CO-O-Ar} \rightarrow \text{Ar-O-CO-O-Ar} + \text{Alk-O-CO-O-Alk} \qquad \text{(Gl. 3)}$$

(Alk = Alkyl, Ar = Aryl)

Die Ausführungen dieser EP 461 274 führen den Fachmann zu dem Schluß, daß man die Umesterung von Phenolen mit Dialkylcarbonaten zwar in bekannter Weise kontinuierlich nach bekannten Verfahren in Kolonnen durchführen kann, daß es aber gleichgültig ist, nach welcher Variante, ob bei hoher oder niedriger Temperatur, im Gleich- oder Gegenstrom, bei niedrigem oder hohem Druck, mit großen oder kleinen Molverhältnissen usw. Kurz man muß daraus schließen, daß bei diesem besonderen Umesterungsproblem keine Möglichkeiten zur Verbesserung und vorteilhafteren Arbeitsweise bestehen.

So können nur die angegebenen Beispiele zur Beurteilung des tatsächlichen Wertes dieser EP herangezogen werden.

Aus diesen Beispielen ist erkennbar, daß bei der Umesterung von Dialkylcarbonaten mit Phenolen selbst bei relativ hohen Temperaturen, unter erhöhtem Druck und sogar bei molaren Dialkylcarbonatüberschüssen von mehr als 3 nur geringe Umsätze im Bereich von 10 bis 15 % (im besten Fall ca. 19 %) und vor allem nur sehr geringe Raumzeitausbeuten bis zu 0,02 kg $\text{l}^{-1}\text{h}^{-1}$ erzielt werden. Das ist verwunderlich, zumal sehr große Kolonnen darunter auch eine 20bödige Kolonne mit 6 m Länge und ca. 300 l Volumen verwendet wurden. Der durch Dialkylcarbonatüberschüsse erzielbare höhere Phenolumsatz muß allerdings aus stöchiometrischen Gründen durch niedrigere Dialkylcarbonatumsätze erkauft werden. Das bedeutet, daß das am Kopf entnommene Dialkylcarbonat nur sehr geringe Mengen Alkohol enthält und daher in einem technischen Verfahren erheblich mehr unumgesetztes Ausgangsprodukt im Kreis gefahren und von den kleinen Mengen Reaktionsalkohol abgetrennt werden muß. Die geringen Raumzeitausbeuten würden bei vorgegebener Produktionsmenge pro Zeiteinheit sehr große Reaktoren und sehr große Destillationskapazitäten notwendig machen.

Die in einer nachgeschalteten zweiten Kolonne betriebene Disproportionierungsreaktion von Alkylarylcarbonaten gemäß Gleichung 3 läuft zwar mit höheren Ausbeuten ab, aber für eine technische Synthese von Diarylcarbonaten ist eine solche Disproportionierung von Alkylarylcarbonaten gegenüber der weiteren Umesterung mit Phenolen als wenig vorteilhaft anzusehen, da nur jedes zweite Alkylarylcarbonatmolekül in das Diarylcarbonat-Endprodukt übergeführt und die andere Hälfte in das Ausgangsdialkylcarbonat zurückgeführt wird.

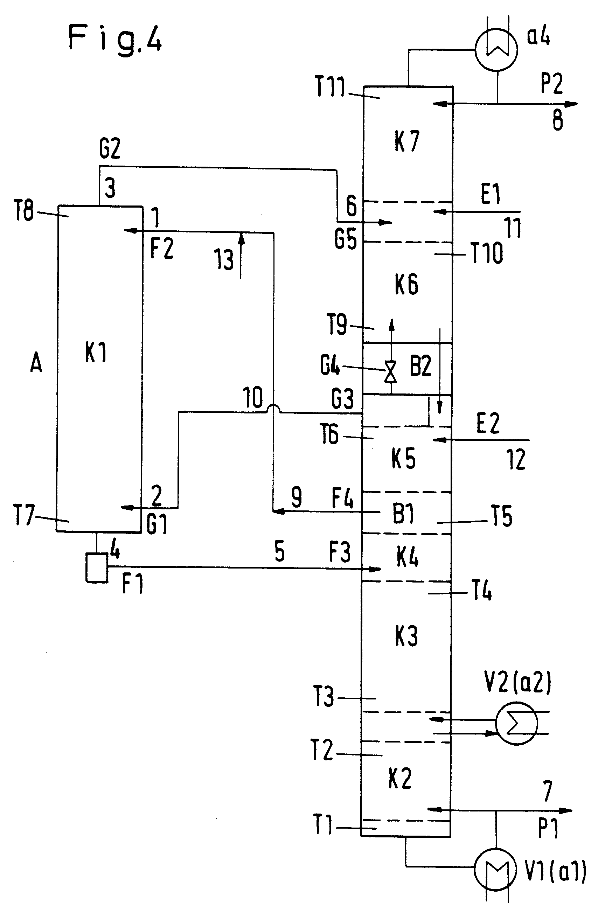

Etwa aus den Beispielen 22 bis 30 der EP 0 461 274, in denen Umsetzungen in zwei hintereinander verschalteten Kolonnen beschrieben werden und die Zusammensetzung des Kopfprodukts aus der zweiten Kolonne als Feedstrom Nr. 6 in Abb. 4 oder 5 genannt ist, wird deutlich, daß trotz Anwesenheit von erheblichen Mengen an Phenolen kein Alkohol in der zweiten Reaktionsstufe gebildet wird und damit der Anteil der zweiten Umesterungsstufe gemäß Gleichung 2 gleich Null ist.

In einem technischen Prozeß zur Herstellung von Diarylcarbonaten, speziell von Diphenylcarbonat aus Dimethylcarbonat und Phenol, ist nicht nur der Phenolumsatz von Bedeutung, sondern auch die Dimethylcarbonatmenge, die zur Erzielung eines bestimmten Phenolumsatzes notwendig ist und der sich daraus ergebende Dimethylcarbonatumsatz. In der Praxis wird man nach einer solchen Verfahrensvariante nur geringe Dimethylcarbonatumsätze und damit geringe Methanolkonzentrationen im Dimethylcarbonat am Kolonnenkopf erreichen können, beispielsweise solche von 5 bis 10 Gew.-% an Methanol. In der EP 461 274 geht man aber nun ohne Einschränkungen von reinem Dimethyl- oder Diethylcarbonat als Edukt aus. Dies ist angesichts der erhaltenen niedrigen Umsätze an Dialkylcarbonaten von nur wenigen Prozent verständlich und sicher unbedingt notwendig, weil aufgrund der ungünstigen Gleichgewichtslage beim Einsatz von alkoholhaltigen Dialkylcarbonaten die Umsätze noch niedriger und damit technisch untragbar würden. Das Methanol aber bildet mit Dimethylcarbonat ein nur mit großem Destillationsaufwand trennbares Azeotrop der Zusammensetzung 70 Gew.-% Methanol und 30 Gew.-% Dimethylcarbonat.

Einen besonders hohen Trennaufwand erfordert jedoch die Entfernung sehr geringer Mengen des Reaktionsmethanols aus dem Dimethylcarbonatproduktstrom, wodurch die Rückführung des nicht umgesetzten Dimethylcarbonats in den Umesterungsprozeß in reiner Form nur unter großem Aufwand erreicht werden kann. Das ist auch von besonderer ökonomischer Bedeutung, da aufgrund nur geringer Dimethylcarbonatumsätze, die während eines Reaktordurchgangs erzielt werden können, die Dimethylcarbonatumlaufmengen sehr groß werden.

Ziel eines verbesserten Umesterungsverfahrens zur Herstellung von Diarylcarbonaten aus Dialkylcarbonaten und Phenolen müßte es also sein, erstens deutliche Mengen Alkohole im Dialkylcarbonat-Eduktstrom tolerierbar zu machen und zweitens die Umesterungsstufe gemäß Gleichung 2, also die Umesterung von Phenol mit Alkylarylcarbonat zu Diarylcarbonat zu begünstigen und die Disproportionierung von Alkylarylcarbonat zurückzudrängen.

Aus dem Massenwirkungsgesetz läßt sich ableiten, daß schon geringe Mengen an Alkoholen mit den schon gebildeten Arylcarbonaten aufgrund des sehr ungünstig liegenden Umesterungsgleichgewichts wieder in Richtung der Edukte abreagieren würden. Daher erscheint es aussichtslos, das oben angegebene erste Ziel zu realisieren. Das haben wohl auch die Autoren der EP 0 461 274 angenommen.

Die Umesterung eines Alkylarylcarbonats mit Phenol zu Diarylcarbonat gemäß Gleichung 2 ist nach den Ergebnissen der EP 461 274 offensichtlich gegenüber der Disproportionierung zweier Alkylarylcarbonatmoleküle gemäß Gleichung 3 benachteiligt oder sogar völlig unterdrückt. Damit ist auch höchst fraglich, ob das zweite Ziel erreicht werden kann. Für eine technische Synthese ist weiter eine Erhöhung der Raum-Zeit-Ausbeuten über die in der EP 461 274 genannten als ein drittes Ziel anzustreben, um die Größe der Apparate zu reduzieren. Auch dafür bietet EP 461 274 keine Lösung.

Überraschenderweise wurde nun gefunden, daß sich die Umesterung von Phenolen mit Dialkylcarbonaten in einem mehrstufigen Prozeß durchführen läßt, in dem sich die gewünschten Ziele realisieren lassen und außerdem eine optimale Energieausnutzung erreicht wird. Dazu wird eine stofflich und energetisch gekoppelte Kombination aus zwei kolonnenartigen Reaktoren benutzt.

Die Erfindung betrifft also ein Verfahren zur Herstellung von Diarylcarbonaten der Formel I

$$Ar^1\text{-}O\text{-}CO\text{-}O\text{-}Ar^1 \tag{I},$$

in der

$Ar^1$ unsubstituiertes Phenyl, mit 1 bis 3 $C_1$-$C_4$-Alkyl-, $C_1$-$C_4$-Alkoxy- und Halogenresten substituiertes Phenyl oder Naphthyl bedeutet

durch Umesterung von aromatischen Hydroxyverbindungen der Formel

$$Ar^1OH \tag{II},$$

in der

$Ar^1$ die gegebene Bedeutung hat

mit 0,1 bis 10 Mol, bevorzugt mit 0,5 bis 2 Mol und besonders bevorzugt mit 0,8 bis 1,2 Mol, Dialkylcarbonaten der Formel

$$R^1\text{-}O\text{-}CO\text{-}O\text{-}R^1 \tag{III},$$

in der

$R^1$ geradkettiges oder verzweigtes $C_1$-$C_6$-Alkyl oder $C_5$-$C_6$-Cycloalkyl bedeutet,

in Gegenwart von an sich bekannten Umesterungskatalysatoren in Kolonnenapparaturen, wie sie für Umesterungsreaktionen bekannt sind, das dadurch gekennzeichnet ist, daß die Reaktion in einer stofflich und energetisch gekoppelten Kombination aus einer Gegenstromkolonne A und einer Reaktionskolonne B, wobei in den genannten Apparaturen Reaktionen und Trennungen parallel laufen, so durchgeführt wird, daß in der Gegenstromkolonne A die aromatischen Hydroxyverbindungen, die mindestens teilweise flüssig der Reaktionskolonne B entnommen wurden, in der Flüssigphase in Gegenwart eines Umesterungskatalysators mit einem gasförmig dazu im Gegenstrom geführten Gemisch aus 100 bis 95 Gew.-Tln. Dialkylcarbonat und 0 bis 5 Gew.-Tln. des zugrundeliegenden Alkohols der Formel

$$R^1OH \qquad\qquad (IV),$$

in der

R$^1$ die gegebenen Bedeutungen hat,

wobei das Gemisch gasförmig der Reaktionskolonne B entnommen wurde und aromatische Hydroxyverbindungen Ar$^1$OH enthalten kann, bei Temperaturen von 100 bis 300°C und Drücken von 0,05 bis 20 bar umgesetzt wird und die in A als Sumpfprodukt anfallenden Gemische aus Alkylarylcarbonaten der Formel

$$Ar^1\text{-}O\text{-}CO\text{-}O\text{-}R^1 \qquad\qquad (V),$$

in der

Ar$^1$ und R$^1$ die oben gegebene Bedeutung besitzen,

aus nicht umgesetzten aromatischen Hydroxyverbindungen, gegebenenfalls aus einer geringen Menge des Dialkylcarbonats und gegebenenfalls aus homogen gelöstem Katalysator in flüssiger Form in den unteren Teil der Reaktionskolonne B und die in A als Kopfprodukte anfallenden gasförmigen Gemische aus den Alkoholen, noch nicht umgesetztem Dialkylcarbonat und aromatischen Hydroxyverbindungen in den oberen Teil der Reaktionskolonne B eindosiert und bei Temperaturen von 100 bis 300°C und Drücken von 0,05 bis 5 bar zu 50 bis >95 % umgesetzt werden, wobei ferner im unteren Teil der Kolonne B Diarylcarbonat als Sumpfprodukt entnommen wird, im mittleren Teil von B und oberhalb der Einspeisung des Sumpfprodukts von A der nach A zurückzuführende flüssige Strom der aromatischen Hydroxyverbindungen entnommen wird, im mittleren Teil von B zwischen der Entnahme der flüssigen aromatischen Hydroxyverbindungen und der Einspeisung des Kopfprodukts von A das nach A zurückzuführende gasförmige Gemisch aus 95 bis 100 Gew.-Tln. Dialkylcarbonat und 0 bis 5 Gew.-Tln. der davon abgeleiteten Alkohole und der aromatischen Hydroxyverbindung entnommen wird und als Kopfstrom von B ein Gemisch von 80 bis 20 Gew.-% der abgeleiteten Alkohole und 20 bis 80 Gew.-% Dialkylcarbonat entnommen wird, wobei umgesetztes oder entnommenes Dialkylcarbonat und umgesetzte aromatische Hydroxyverbindungen durch Einspeisung in A oder B ergänzt werden.

Erfindungsgemäß herstellbare Diarylcarbonate sind demnach Verbindungen der Formel

$$Ar^1\text{-}O\text{-}CO\text{-}O\text{-}Ar^1 \qquad\qquad (I),$$

in der

Ar$^1$ die obige Bedeutung besitzt, beispielsweise Diphenylcarbonat, die isomeren Bis-tolylcarbonate, die isomeren Bis-(ethylphenyl)carbonate, die isomeren Bis-(chlorphenyl)carbonate, die isomeren Bis-(bromphenyl)carbonate, die isomeren Bis-(methoxyphenyl)carbonate, Bis-(1-naphthyl)carbonat, Bis-(2-naphthyl)carbonat und Bis-(1,6-dimethylphenyl)carbonat, bevorzugt Diphenylcarbonat und Bis-(tolyl)carbonat und besonders bevorzugt Diphenylcarbonat.

Einsetzbare aromatische Hydroxyverbindungen sind demnach solche der Formel

$$Ar^1OH \qquad\qquad (II),$$

in der

Ar$^1$ die obige Bedeutung hat.

Genannt seien beispielsweise Phenol, die isomeren Kresole, die isomeren Ethylphenole, die isomeren Chlorphenole, die isomeren Bromphenole, die isomeren Methoxyphenole, die isomeren Naphthole und Xylenole. Besonders bevorzugt einsetzbar sind Phenol und die Kresole. Ganz besonders bevorzugt einsetzbar ist Phenol selbst.

Erfindungsgemäß werden Dialkylcarbonate der Formel

$$R^1\text{-}O\text{-}CO\text{-}O\text{-}R^1 \qquad\qquad (III)$$

eingesetzt, in der R$^1$ die obige Bedeutung besitzt, beispielsweise Dimethylcarbonat, Diethylcarbonat, Dipropylcarbonat, Dibutylcarbonat, Dihexylcarbonat, Dicyclohexylcarbonat und Dicyclopentylcarbonat. Besonders bevorzugt kann Dimethyl- und Diethylcarbonat und ganz besonders bevorzugt Dimethylcarbonat verwendet werden.

Geradkettiges oder verzweigtes $C_1$-$C_6$-Alkyl ist beispielsweise Methyl, Ethyl, Propyl, Isopropyl, Butyl, Isobutyl, Pentyl oder Hexyl.

Geradkettiges oder verzweigtes $C_1$-$C_4$-Alkoxy ist beispielsweise Methoxy, Ethoxy, Propoxy, Isopropoxy, Butoxy oder Isobutoxy.

Halogen ist beispielsweise Fluor, Chlor oder Brom, bevorzugt Fluor oder Chlor, besonders bevorzugt Chlor.

Der als Kolonne A bezeichnete Reaktor wird hierbei überwiegend in Gegenstromfahrweise betrieben, d.h. eine vom Kopf der Kolonne herabströmende flüssige aromatische Hydroxyverbindung wird auf der überwiegenden Kolonnenstrecke mit gasförmig entgegengeführtem Dialkylcarbonat umgesetzt, wobei beide Edukte einem zweiten Reaktor B an geeigneter Stelle entnommen werden. Das dabei gebildete Alkylarylcarbonat wird am Fuß der Kolonne A, gegebenenfalls zusammen mit nicht umgesetzter aromatischer Hydroxyverbindung und Dialkylcarbonat, der leichtflüchtige Reaktionsalkohol im Gemisch mit nicht umgesetztem Dialkylcarbonat und der aromatischen Hydroxyverbindung am Kopf der Kolonne A entnommen. Es wird in einer bevorzugten Fahrweise darauf verzichtet, wesentliche Anteile des noch nicht umgesetzten Phenols durch Verdampfung am Fuß der Kolonne bzw. wesentliche Anteile des noch nicht umgesetzten Dialkylcarbonats durch Rückfluß am Kopf der Kolonne zurück in den Reaktor zu schaffen, so daß der Energieeintrag bzw. die Energieentnahme an dieser Stelle des Verfahrens relativ gering bleibt. Die am Kopf bzw. am Fuß der Kolonnne A anfallenden Produkt/Edukt-Ströme werden an geeigneter Stelle in den kolonnenartigen Reaktor B geführt.

In diesem Reaktor B wird das im Reaktor A erzeugte Alkylarylcarbonat im Sinne einer "Reaktionsdestillation" weiter umgesetzt. Die für eine "Reaktionsdestillation" im Sinne der Erfindung wesentlichen Merkmale sind dabei die folgenden: Das Alkylarylcarbonat wird durch einen speziell gewählten Temperaturgradienten weitgehend daran gehindert, die Reaktionszone des Reaktors nach oben oder nach unten zu verlassen. Die leichtflüchtigen Reaktionsprodukte, hier Reaktionsalkohol, Dialkylcarbonat und überschüssiges Phenol, werden in den oberen und mittleren Bereich der Kolonne B transportiert, das schwerflüchtige Reaktionsprodukt, hier das Diarylcarbonat, wird am Fuß der Kolonne über (6) entnommen.

Im Reaktor B laufen gleichzeitig noch weitere Trennprozesse ab, die es ermöglichen, dem Reaktor an voneinander getrennten Stellen die noch nicht umgesetzten aromatischen Hydroxyverbindungen, sowie alkoholarme und alkoholreiche Dialkylcarbonat/Alkohol-Gemische zu entnehmen, wobei die alkoholreiche Dialkylcarbonat-Fraktion am Kopf der Kolonne B als Leichtsiederprodukt entnommen wird. Die alkoholarmen Dialkylcarbonat-Fraktionen und die aromatischen Hydroxyverbindungen werden in den Reaktor A zurückgeführt.

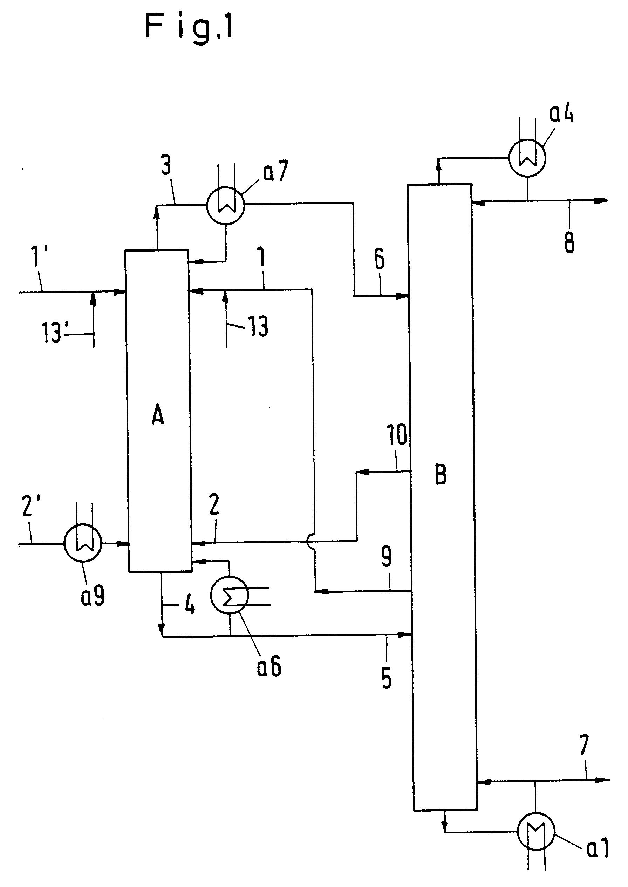

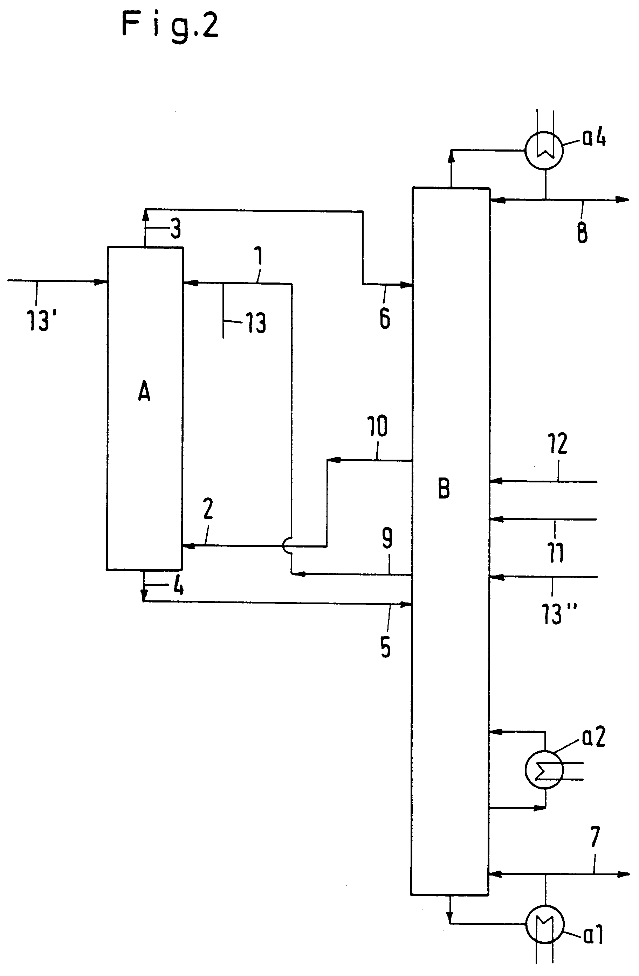

Die für die Kolonne A bzw. für die Kolonne B notwendigen Edukte werden also wechselweise aus dem jeweils gekoppelten Reaktor bezogen. Die Edukte, die durch Umsetzung oder durch Austragen zusammen mit den Produkten dem Gesamtprozeß verloren gehen, können entweder direkt in den Reaktor A im Sinne der oben angegebenen Gegenstromfahrweise oder bevorzugt in den mittleren oder oberen Teil des Reaktors B eingespeist werden (siehe Fig. 1 und 2).

Der überwiegende Teil der Energie für den Gesamtprozeß wird im unteren Teil des Reaktors B eingebracht, dient dort der Umsetzung zum Diarylcarbonat, wird mit der überschüssigen, zurückdestillierenden aromatischen Hydroxyverbindung in den mittleren und oberen Bereich der Kolonne gebracht und betreibt dort die oben angegebenen Trennungen. Gleichzeitig kann die Energie aus Kolonne B zum Betrieb der Kolonne A über die vom Reaktor B nach A zurückgeführten Eduktströme genutzt werden.

Mit der oben beschriebenen, auf das spezielle Umesterungsproblem angepaßten Verfahrensweise in zwei stofflich und energetisch gekoppelten kolonnenartigen Reaktoren erreicht man ohne Isolierung der Alkylarylcarbonat-Zwischenprodukte gleichzeitig hohe Umsätze der aromatischen Hydroxyverbindungen und der Dialkylcarbonate. Die am Füße bzw. am Kopf des Reaktors B entnommenen Produkte sind schon im hohen Maß angereichert und daher leicht zu reinigen bzw. direkt in angegliederte gekoppelte Prozesse, etwa zur erneuten Herstellung von Dimethylcarbonat, rückführbar. Beispielsweise erhält man am Kopf des Reaktors B Methanol-Dimethylcarbonat-Gemische, die beinahe schon die Zusammensetzung des Methanol-Dimethylcarbonat-Azeotrops von 30:70 Gew.-% besitzen und ohne weitere Anfkonzentrierung in einen Umesterungsprozeß zur Herstellung von Dimethylcarbonat eingesetzt werden können. Außerdem ist es möglich, alkoholhaltige Dialkylcarbonate in den erfindungsgemäßen Prozeß einzusetzen. Die Energie wird im wesentlichen an einer Stelle des Prozesses, nämlich am unteren Ende des Reaktors B eingebracht und auch im wesentlichen wieder an einer Stelle, am Kopf der Kolonne B entnommen und intern mehrmals für verschiedene Prozeßschritte genutzt, d.h. mit dem erfindungsgemäßen Verfahren ist eine optimale Energieausnützung möglich.

Der als "Gegenstromkolonne" bezeichnete Reaktor A stellt im einfachsten Fall ein isotherm beheiztes oder bevorzugt ein adiabatisch isoliertes, mit üblichen, für Destillationen zu verwendenden Füllkörpern, Packungen oder Kolonneneinbauten versehenes Rohr dar.

Die Kolonne kann am unteren Ende ein bei höheren Temperaturen arbeitendes Abtriebsventil enthalten, in dem eine weitgehende bis vollständige Separierung des eindosierten Dialkylcarbonats von der herabrieselnden Flüssigphase erfolgt, wobei es wieder dampfförmig in den Umesterungsbereich der Kolonne geführt wird. Weiterhin kann die Kolonne am oberen Teil ein Verstärkerteil beinhalten, das mitverdampftes Phenol oder Alkylphenylcarbonat von den leichtsiedenden Reaktionsalkoholen, bzw. Dialkylcarbonaten abtrennt und flüssig in den Umesterungsteil der Kolonne zurückführt.

In einer bevorzugten Fahrweise kann jedoch an diesen Stellen auf solche Verstärker-bzw. Abtriebsteile verzichtet werden.

Im Rahmen des erfindungsgemäßen Verfahrens ist es zweckmäßig, die für die Reaktion notwendige Energie nicht nur über eine Mantelbeheizung oder über andere Wärmetauscher, sondern sowohl mit dem eingesetzten Phenol, als auch mit dem gasförmig eindosierten Dialkylcarbonat einzubringen. Die Verdampfungsenergie für das Dialkylcarbonat kann ggf. über einen separaten oder einen in der Säule integrierten Verdampfer aufgebracht werden. Weiterhin können in die Säule zum Ausgleich von Reaktionswärmen innen- oder außenliegende Wärmetauscher eingebaut werden. Die Kolonne kann über die gesamte Länge entweder gleiche Temperatur, oder einen Temperaturgradienten aufweisen. Die Auslegung des Umesterungs-, des Abtriebs-und des Verstärkerteils kann vom Fachmann vorgenommen werden.

Die zu verwendenden Füllkörper bzw. geordnete Packungen sind die für Destillationen an sich üblichen, wie sie beispielsweise in Ullmann's Encyclopädie der Technischen Chemie, 4. Aufl., Bd. 2, S. 528 ff. oder in den Firmenschriften der in Frage kommenden Apparatebaufirmen beschrieben sind. Beispielsweise seien genannt: Raschig- oder Pallringe, Berl-, Intalox- oder Torussättel, Interpackkörper aus verschiedenen Materialien, wie Glas, Steinzeug, Porzellan, Edelstahl, Kunststoff, die insbesondere bei der Verwendung von Metall, gewebe- oder maschenartig verarbeitet sein können. Bevorzugt sind Füllkörper und geordnete Packungen, die eine große Oberfläche, eine gute Benetzung sowie ausreichende Verweilzeit der Flüssigphase aufweisen.

Diese sind beispielsweise Pall- und Novolaxringe, Berlsättel, BX-Packungen, Montz-Pak, Mellapak, Melladur, Kerapak und CY-Packungen.

Für das erfindungsgemäße Verfahren, insbesondere für den Reaktor A, sind jedoch nicht nur Füllkörperkolonnen geeignet, sondern auch solche mit festen Einbauten. Geeignet sind allgemein Bodenkolonnen, z.B. solche mit Sieb-, Glocken-, Ventil-, Tunnel- und Zentrifugalböden, die wiederum in unterschiedlichen Ausführungen vorliegen können. Hierunter sind solche mit Glocken- bzw. Ventilböden mit hohen Verweilzeiten bei gutem Stoffaustausch bevorzugt, beispielsweise Glockenbödenkolonnen mit hohen Überlaufwehren, wie sie beispielsweise in DE-OS 2503195 beschrieben sind.

Die theoretische Bodenzahl der als Reaktor A zu verwendenden Kolonne beträgt 3 bis 50, bevorzugt 3 bis 30 und besonders bevorzugt 5 bis 20 Böden, der Flüssigkeits-Hold up 1 bis 80 %, bevorzugt 5 bis 75 % und besonders bevorzugt 10 bis 50 % des Kolonneninnenvolumens. Die genauere Auslegung des Umesterungs- und des gegebenenfalls zu verwendenden Abtriebs- und des Verstärkerteils kann vom Fachmann vorgenommen werden.

Die Kolonne A wird innerhalb des Gesamtprozesses (Fig. 1 bis 3) so betrieben, daß man in die obere Hälfte, bevorzugt in das obere Drittel und besonders bevorzugt auf dem obersten Boden oder am Kopf der Packung, einen aus der Kolonne B stammenden Phenolstrom über (1), bevorzugt mit der an dieser Stelle der Kolonne herrschenden Temperatur, flüssig eindosiert. Dieser Phenolstrom kann gegebenenfalls geringe Konzentrationen an Dialkylcarbonat und Alkohol, die der Löslichkeit dieser Komponenten im entsprechenden Phenol bei der gegebenen Temperatur entsprechen, enthalten. Die gewünschte Temperatur kann mit Hilfe separater Wärmetauscher eingestellt werden. Bevorzugt wird jedoch das Phenol ohne weitere Erwärmung oder Abkühlung aus der Kolonne B bei (9) entnommen und bei (1) der Kolonne A zugeführt.

In die untere Hälfte der Kolonne A, bevorzugt oberhalb einer gegebenenfalls vorhandenen Abtriebszone, wird über (2) ein Dialkylcarbonatstrom, der bei (10) aus der Kolonne B entnommen wird, in der Regel in Dampfform, mit Temperaturen von 120 bis 220°C eindosiert. Dieser Dialkylcarbonatstrom enthält 0 bis 5 Gew.-%, bevorzugt 0,1 bis 3 Gew.-% und besonders bevorzugt 0,2 bis 2 Gew.-%, des korrespondierenden Alkohols und erhebliche Mengen eines Phenols.

Nach Passieren der Umesterungszone wird der Reaktionsalkohol, gegebenenfalls nach Durchlaufen einer Verstärkerzone, am Kopf der Kolonne bei (3) entnommen. Er enthält im allgemeinen noch überschüssiges oder nicht umgesetztes Dialkylcarbonat und bei nicht vorhandenem Verstärkerteil auch größere Mengen der aromatischen Hydroxyverbindung. Dieser Kopfstrom wird bevorzugt ohne vorherige Kondensation gasförmig bei (6) in die Kolonne B geführt.

Nach Passieren der Umesterungszone und eines gegebenenfalls vorhandenen Abtriebsteils tritt am Füße der Kolonne A bei (4) ein Gemisch von Alkylarylcarbonat, mit überschüssigem oder nicht umgesetztem Phenol, mit gegebenenfalls geringen Mengen von schon gebildetem Diarylcarbonat, mit gegebenenfalls löslichen Katalysatoren und bei einer Fahrweise ohne Abtriebsteil auch mit Dialkylcarbonat aus. Das Sumpfprodukt wird direkt über (5) in die zweite Umesterungskolonne B eingespeist.

Der Katalysator wird bevorzugt oberhalb oder auf gleicher Höhe wie die Phenoldosierung (1) in Kolonne A bei (13') in gelöster oder suspendierter Form, entweder mit geringen Mengen Phenol, mit Reaktionsalkohol oder in einem systemfremden, geeigneten inerten Lösungsmittel eingebracht. Besonders bevorzugt wird der Katalysator mit dem aus der Kolonne B stammenden flüssigen Phenolstrom in die Kolonne A eingespeist, wobei der Katalysator beispielsweise bei (13) seitlich in den Phenolstrom eingebracht werden kann. Im Falle der Verwendung heterogener Katalysatoren können diese im Gemisch mit den genannten Füllkörpern, in geeigneter Form anstelle von Füllkörpern oder als Schüttung auf eingebaute Kolonnenböden eingesetzt werden.

Das Molverhältnis der eingesetzten Edukte in der Kolonne A variiert von 0,1 bis 10 Mol, bevorzugt von 0,2 bis 5 Mol und besonders bevorzugt von 0,5 bis 3 Mol Dialkylcarbonat pro Mol eingesetztem Phenol.

Die Umesterung im Reaktor A kann bei Temperaturen von 100 bis 300°C, bevorzugt bei Temperaturen von 120 bis 250°C und besonders bevorzugt bei Temperaturen von 140 bis 230°C in der Kolonne durchgeführt werden. Der anliegende geringe Temperaturgradient liegt im angegebenen Temperaturbereich und steigt vom Kolonnenkopf in Richtung Kolonnenfuß. Dabei muß gewährleistet sein, daß die Reaktionstemperatur im Umesterungsbereich nicht oberhalb der Verdampfungstemperatur des eingesetzten Phenols liegt. Es ist deshalb von Vorteil, die erfindungsgemäße Umesterung nicht nur bei Normaldruck, sondern auch bei erhöhtem oder erniedrigtem Druck von 50 mbar bis zu 20 bar durchzuführen. Ein bevorzugter Druckbereich liegt zwischen 0,2 und 12 bar, ein besonders bevorzugter Druckbereich liegt zwischen 0,5 und 10 bar.

Die Raum-Zeit-Belastung der Kolonne A liegt bei 0,025 bis 3 g Gesamtmenge der Reaktionsteilnehmer pro ml wirksames Kolonnenvolumen pro Stunde, bevorzugt bei 0,05 bis 3 g/ml/h, besonders bevorzugt bei 0,1-2 g/ml/h; das wirksame Kolonnenvolumen ist hierbei das der Füllkörperpackung oder das Volumen, in welchem sich feste Einbauten befinden.

Der als "Reaktionskolonne" bezeichnete Reaktor B (Fig. 1 bis 3) besteht aus einem kolonnenartigen Rohr, dem ein Temperaturprofil angelegt wird, das von oben nach unten gesehen ansteigend einen Temperaturbereich von 50 bis 320°C, bevorzugt von 60 bis 300°C, umfaßt. Zur Einstellung der Temperaturgradienten in den einzelnen Abschnitten des kolonnenartigen Reaktors können diese Abschnitte mit einer Isolierung bzw. einer Thermostatisierung versehen werden. Die Thermostatisierung kann hierbei je nach Bedarf eine Heizung oder eine Kühlung darstellen (a2 und a3). Der Reaktor B kann in verschiedenen Abschnitten seiner Gesamtlänge, entsprechend der Gas- und Flüssigkeitsbelastungen und der benötigten Verweilzeiten, aufgeweitet oder verengt sein.

Das Kolonnenrohr des Reaktors B kann mit üblichen, für Destillationen zu verwendenden Füllkörpern oder Packungen gefüllt sein und außerdem feste Einbauten besitzen, wobei in den einzelnen Bereichen der Kolonne bevorzugt verschiedenartige Füllkörper, Packungen oder feste Einbauten eingesetzt werden können. Die zu verwendenden Füllkörper bzw. geordnete Packungen sind die für Destillationen an sich üblichen, wie sie beispielsweise in Ullmanns Encyclopädie der Techn. Chemie, 4. Aufl., Bd. 2, S. 528ff oder in den Firmenschriften der in Frage kommenden Apparatebaufirmen beschrieben sind. Beispielsweise seien genannt: Raschig- oder Pallringe, Berl-, Intalex- oder Torussättel, Interpackkörper aus verschiedenen Materialien, wie Glas, Steinzeug, Porzellan, Edelstahl, Kunststoff, die insbesondere bei der Verwendung von Metall, gewebe- oder maschenartig verarbeitet sein können. Bevorzugte Füllkörper oder Packungen sind beispielsweise Pall- und Novolaxringe, Berlsättel, BX-Packungen, Montz-Pak, Metallpak, Melladur, Kerapak und CY-Packungen.

Für den Reaktor B sind jedoch nicht nur Füllkörper geeignet, sondern auch feste Einbauten. Geeignet sind allgemein solche, die in Bodenkolonnen üblich sind, z.B. Sieb-, Glocken-, Ventil-, Tunnel- und Zentrifugalböden, die wiederum in unterschiedlichen Ausführungen vorliegen können. Hierunter sind Glocken- bzw. Ventilböden mit hohen Verweilzeiten bei gutem Stoffaustausch, beispielsweise Glockenböden mit hohen Überlaufwehren bevorzugt. Für den Reaktionsbereich der Kolonne B sind feste Einbauten bevorzugt, für den Bereich, in dem Trennungen stattfinden, dagegen Füllkörper und feste Packungen.

Am unteren Ende der Kolonne B sind ein oder mehrere, gegebenenfalls durch adiabatisch isolierte Kolonnenteile getrennte, Verdampfer angeordnet. Diese Verdampfer können innerhalb oder bevorzugt außerhalb der Kolonne angeordnet sein. In einer technischen Ausführung der Erfindung werden in der Technik übliche Apparate wie Umlaufverdampfer, Fallfilmverdampfer und Wendelrohrverdampfer verwendet.

Oberhalb der Verdampferzone in dem als "Reaktionszone" bezeichneten Bereich werden bevorzugt feste Einbauten und besonders bevorzugt solche mit hohem Flüssigkeits-Hold Up benutzt beispielsweise Glockenböden mit hohen Überlaufwehren. Die theoretische Bodenzahl in diesem Bereich beträgt 2 bis 50, bevorzugt 2 bis 25 und besonders bevorzugt 2 bis 15. Der Flüssigkeits-Hold Up in diesem Bereich beträgt 5 bis 80 %, bevorzugt 10 bis 75 % und besonders bevorzugt 15 bis 50 % des Innenvolumens der Einbauten.

Wiederum oberhalb dieses Bereiches ist die Kolonne mit weiteren, im besonderen Maße für destillative Stofftrennungen geeigneten Füllkörpern oder Einbauten ausgestattet. Am oberen Ende der Kolonne B ist ein Verstärkerteil angeordnet, mit dem ein gezielter Rücklauf auf die Kolonne einstellbar ist.

In einer bevorzugten Ausführungsweise der Erfindung wird der Hauptteil der für den Gesamtprozeß erforderlichen Energie durch die am unteren Ende der Kolonne B angeordneten Verdampfer in den Prozeß eingebracht und durch den am oberen Ende des Reaktors B angeordneten Verstärkerteil wird der Hauptteil der Überschußenergie wieder entnommen. Dieser wird intern für Trennungen und die in Kolonne A und B ablaufenden Reaktionen genutzt.

Die Kolonne B wird innerhalb des Gesamtprozesses (Fig. 1 bis 3) so betrieben, daß man in den mittleren Teil der Kolonne oberhalb des als "Reaktionszone" bezeichneten Bereichs über die Eindosierung (5) einen aus der Kolonne A bei (4) entnommenen Strom aus Alkylarylcarbonat und aromatischer Hydroxyverbindung, der gegebenenfalls geringe Mengen Diarylcarbonat, Dialkylcarbonat und einen Umesterungskatalysator enthalten kann, flüssig eindosiert. Dieser Strom durchläuft die "Reaktionszone" und wird dort teilweise in Diarylcarbonat verwandelt und die noch nicht umgesetzten Reaktanden mit Hilfe der beschriebenen Verdampfer gasförmig zurück in die mittleren und oberen Teile der Kolonne B transportiert. Diese kondensieren dort und setzen sich erneut zum Diarylcarbonatendprodukt um. Das Diarylcarbonatendprodukt wird als höchstsiedende Reaktionskomponente im Sumpfbereich der Kolonne angereichert und dort über (7) zusammen mit gegebenenfalls homogen gelöstem Katalysator und geringen Mengen Alkylarylcarbonat und aromatischer Hydroxyverbindung ausgespeist. Der zugehörige Sumpfumlauferhitzer ist (a1).

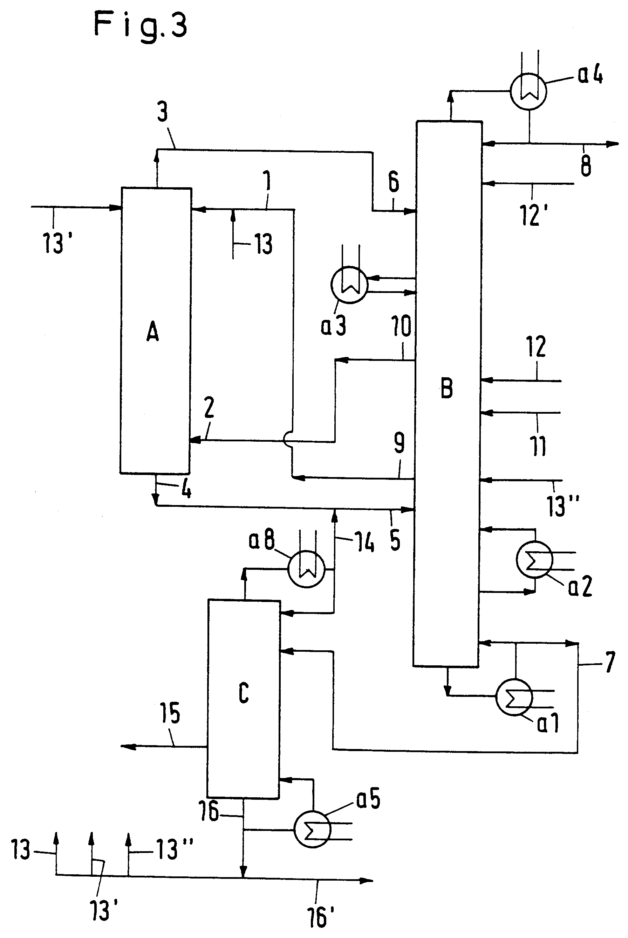

In die obere Hälfte, bevorzugt das obere Drittel der Kolonne B, wird über (6) ein als Kopfprodukt der Kolonne A bei (3) entnommener, oben beschriebener Produktstrom, bevorzugt gasförmig, eindosiert. Dieser Produktstrom wird im oberen Teil der Kolonne B in (i) Gemische aus 80 bis 20 Gew.-% eines Alkohols der Formel (IV) und 20 bis 80 Gew.-% eines Dialkylcarbonats der Formel (III) und bevorzugt in Gemische aus 30 bis 70 Gew.-% eines Alkohols der Formel (IV) und 70 bis 30 Gew.-% eines Dialkylcarbonats der Formel (III) und (ii) Gemische aus 95 bis 100 Gew.-% Dialkylcarbonat (III) und 0 bis 5 Gew.-% eines Alkohols der Formel (IV) und bevorzugt in Gemische aus 97 bis 99,8 Gew.-% (III) und 3 bis 0,2 Gew.-% (IV) aufgespalten. Zusätzlich zu der angegebenen Zusammensetzung und über die 100 %-Marke hinaus enthält das Gemisch (ii) stets auch die aromatische Hydroxyverbindung. Zu dieser Trennung wird ein Kolonnenbereich mit 10 bis 50 theoretischen Böden und bevorzugt mit 10 bis 30 theoretischen Böden benötigt, wobei die Trennung durch einen gezielt einstellbaren Rücklauf beeinflußt werden kann. Der alkoholreichere Teilstrom (i) wird über Kopf bei (8) entnommen (Kondensator a4) und kann entweder einer weiteren Aufarbeitung oder gegebenenfalls bei geeigneter Alkoholkonzentration direkt einem vorgeschalteten Umesterungsprozeß zur Herstellung von Dialkylcarbonat zugeführt werden. Den alkoholarmen Teilstrom (ii) entnimmt man über (10) unterhalb des angegebenen Trennbereichs und führt ihn, wie oben beschrieben, gasförmig bei (2) zurück in die Kolonne A. Die Energie für die oben beschriebene destillative Trennung sowie für die Überhitzung des Gasstroms (10) zu (2) wird im wesentlichen durch das vom Fuß der Kolonne B hochdestillierende und im mittleren Bereich der Kolonne B wieder kondensierende Phenol und Alkylarylcarbonat geliefert. Gegebenenfalls kann zur Unterstützung dieser Vorgänge ein weiterer Wärmetauscher im mittleren Bereich der Kolonne dienen (a2 bzw. a2 und a3 in Fig. 2 und 3).

Das kondensierende Phenol der Formel (II) wird an geeigneter Stelle (9) der Kolonne B unterhalb der Entnahmestelle des Dialkylcarbonatstroms (10) in flüssiger Form, mit Temperaturen knapp unterhalb seines Siedepunkts entnommen und bei (1), wie oben beschrieben, in flüssiger Form in die Kolonne A zurückgeführt. Zur Auftrennung dieses Phenolstroms wird ein Kolonnenteil mit 2 bis 20, bevorzugt 3 bis 10 Böden, benötigt. Es kann außerdem zweckmäßig sein, den Reaktor zwischen der Eindosierstelle (6) und der Gasentnahmestelle (10) gasseitig zu trennen und an dieser Stelle der Kolonne B nur einen Flüssigkeitsstrom von oben nach unten zu ermöglichen, wobei der Gas- und Energiestrom dann mit Hilfe einer regelbaren Dampfteilung teilweise über (10) bis (2), in die Kolonne A und über (3) bis (6) in den oberen Teil der Kolonne B geführt wird. Dies kann beispielsweise durch einen speziellen Zwischenboden zwischen (9) und (10) ermöglicht werden.

In einer besonderen Fahrweise (Fig. 3) kann das im wesentlichen aus Diarylcarbonat bestehende Rohproduktgemisch (7) in einem nachgeschalteten separaten Reaktor C bei Drücken von 0,05 bis 1,0 bar noch weiter umgesetzt und aufgetrennt werden, wobei im Seitenstrom dieser Kolonne über (15) reines Diarylcarbonat, am Fuß der Kolonne ein Diarylcarbonat-haltiger Katalysatorsumpf (16) und über Kopf bei (14) die nicht umgesetzten Edukte bzw. gebildeten leichtsiedenden Produkt entnommen werden. Der Katalysatorsumpf (16) läßt sich an geeigneter Stelle in den Prozeß zurückführen, z.B. in den Reaktor A an oben angegebener Stelle (13, 13') oder in den Reaktor B (13"). Bei teilweiser Desaktivierung ist es natürlich auch möglich, einen Teil des Katalysatorsumpfs zu entnehmen (16') und den entfernten Teil an Katalysator an geeigneter Stelle durch frischen Katalysator zu ersetzen. Das als Kopfprodukt der Kolonne C anfallende Gemisch läßt sich über den Produktstrom (4) nach (5), gegebenenfalls nach Angleichung des Drucks in den Umesterungsprozeß zurückführen.

Bei der Verwendung von homogen gelösten oder suspendierten Katalysatoren werden im wesentlichen die schon in der Kolonne A wirksamen, im Sumpfprodukt (4) enthaltenen Katalysatoren bei (5) in die Kolonne B eingespeist und dadurch in der Reaktorzone von B wirksam. Darüber hinaus besteht aber auch die Möglichkeit, an einer Stelle unterhalb der Dialkylcarbonat-Entnahmestelle (10) zusätzlichen Katalysator derselben Art oder einen zweiten Katalysator einzuspeisen (13"). Beim Einsatz von heterogenen Katalysatoren können diese im Gemisch mit den genannten Füllkörpern, in geeigneter Form anstelle von Füllkörpern oder in bevorzugter Weise als Schüttung auf eingebauten Kolonnenböden eingesetzt werden.

Die Reaktion in der Kolonne B wird bevorzugt bei einem Druck zwischen 50 mbar und 5 bar, bevorzugt zwischen 0,1 und 3 bar, besonders bevorzugt zwischen 0,2 und 2 bar und ganz besonders bevorzugt bei Umgebungsdruck, durchgeführt. Die Temperaturen in dem als Reaktionszone bezeichneten Bereich der Kolonne B liegen zwischen 100 und 300°C, bevorzugt zwischen 120 und 280°C und besonders bevorzugt zwischen 140 und 260°C.

Die zu Produkten umgesetzten und somit verbrauchten oder zusammen mit den Produkten über (7) oder (8) aus dem Verfahren ausgeschleusten Edukte Dialkylcarbonat bzw. Phenol können entweder in die Kolonne A im Sinne einer Gegenstromumesterung eingespeist werden, wobei die Phenole in flüssiger Form am oberen Ende der Kolonne als (1') und die Diarylcarbonate gasförmig am Fuß der Kolonne A als (2') eingebracht werden. Bevorzugte Eindosierstellen für die Phenole (11) und Dialkylcarbonate (12) liegen jedoch im mittleren und oberen Bereich der Kolonne B, wobei es an dieser Stelle auch möglich ist, außer reinem Dialkylcarbonat und Mischungen mit der weiter oben angegebenen Menge von 0 bis 5 Gew.-% des zugrundeliegenden Alkohols Mischungen aus Dialkylcarbonaten und Alkoholen mit bis zu 20 Gew.-%, bevorzugt bis zu 10 Gew.-% an Alkohol einzusetzen. Es ist sogar möglich, Mischungen aus Dialkylcarbonaten und zu 60 Gew.-% des korrespondierenden Alkohols einzusetzen, wenn diese in die Kolonne B an einer Stelle (12') oberhalb der Dampfeinspeisung (6) aus der Kolonne A eindosiert werden, wie dies in Fig. 3 gezeigt ist. Bevorzugte Mengen an Alkohol sind 0,5 bis 20 Gew.-%, besonders bevorzugt 1 bis 10 Gew.-%, bezogen auf das Gesamtgewicht des Gemisches.

In Fig. 1 und Fig. 3 sind (a6) und (a5) übliche Sumpfumlauferhitzer für die Kolonnen A bzw. C; (a7) und (a8) sind Kondensatoren mit Rückflußteiler an den Kolonnen A bzw. C; (a9) ist ein Verdampfer für Dialkylcarbonat, das bei (2') in die Kolonne A eingespeist wird (Fig. 1).

Fig. 4 zeigt in Ergänzung zu Fig. 2 weitere Details, die im Rahmen der Ausführungsbeispiele erläutert werden.

Für alle erfindungsgemäßen Reaktionsschritte können die gleichen Katalysatoren eingesetzt werden. Diese sind aus der Literatur bekannte Umesterungskatalysatoren für die Dialkylcarbonat-Phenol-Umesterung, wie z.B. Hydride, Oxide, Hydroxide, Alkoholate, Amide und andere Salze von Alkali- und Erdalkalimetallen (US 3.642.858; US 3.803.201; EP 1082), wie von Lithium, Natrium, Kalium, Rubidium, Caesium, Magnesium und Calcium, bevorzugt Lithium, Natrium, Kalium, Magnesium und Calcium und besonders bevorzugt Lithium, Natrium und Kalium. Salze der Alkali- und Erdalkalimetalle können auch solche von organischen oder anorganischen Säuren sein, wie von Essigsäure, Propionsäure, Buttersäure, Benzoesäure, Stearinsäure, Kohlensäure (Carbonate oder Hydrogencarbonate), Phosphorsäure, Blausäure, Rhodanwasserstoffsäure, Borsäure, Zinnsäure, $C_1$-$C_4$-Stannonsäuren oder Antimonsäure. In bevorzugter Weise kommen als Verbindungen der Alkali-und Erdalkalimetalle die Oxide, Hydroxide, Alkoholate, Acetate, Propionate, Benzoate, Carbonate und Hydrogencarbonate in Frage, in besonders bevorzugter Weise werden Hydroxide, Alkoholate, Acetate, Benzoate oder Carbonate eingesetzt.

Die genannten Alkali- oder Erdalkalimetallverbindungen werden in Mengen von 0,001 bis 2 Gew.-%, bevorzugt 0,005 bis 0,9 Gew.-% und besonders bevorzugt 0,01 bis 0,5 Gew.-%, bezogen auf das umzusetzende Reaktionsgemisch, eingesetzt.

Weitere erfindungsgemäß einsetzbare Katalysatoren sind Lewis-saure Metallverbindungen wie $AlX_3$, $TiX_3$, $UX_4$, $TiX_4$, $VOX_3$, $VX_5$, $ZnX_2$, $FeX_3$ und $SnX_4$, worin X für Halogen, Acetoxy, Alkoxy oder Aryloxy steht (DE-OS 2 528 412, 2 552 907), beispielsweise Titantetrachlorid, Titantetraphenoxid, Titantetraethoxid, Titantetraisopropylat, Titantetradodecylat, Zinntetraisooctylat und Alummiumtriisopropylat, weiterhin zinnorganische Verbindungen der allgemeinen Formel $(R^{11})_{4-x}$-Sn(Y)$_x$, in der Y für einen Rest $OCOR^{12}$, OH oder OR steht, wobei $R^{12}$ $C_1$-$C_{12}$-Alkyl, $C_6$-$C_{12}$-Aryl oder $C_7$-$C_{13}$-Alkylaryl bedeutet und $R^{11}$ unabhängig von $R^{12}$ die Bedeutung von $R^{12}$ hat und x eine ganze Zahl von 1 bis 3 bedeutet, Diälkylzinnverbindungen mit 1 bis 12 C-Atomen im Alkylrest oder Bis-(trialkylzinn)verbindungen, beispielsweise Trimethylzinnacetat, Triethylzinnbenzoat, Tributylzinnacetat, Triphenylzinnacetat, Dibutylzinndiacetat, Dibutylzinndilaurat, Dioctylzinndilaurat, Dibutylzinnadipinat, Dibutyldimethoxyzinn, Dimethylzinnglykolat, Dibutyldiethoxyzinn, Triethylzinnhydroxid, Hexaethylstannoxan, Hexabutylstannoxan, Dibutylzinnoxid, Dioctylzinnoxid, Butylziuntriisooctylat, Octylzinntriisooctylat, Butylstannonsäure und Octylstannonsäure in Mengen von 0,001 bis 20 Gew.-% (EP 879, EP 880, EP 39 452, DE-OS 3 445 555, JP 79/63023), polymere Zinnverbindungen der Formel -[-R,$R^{11}$Sn-O-]-, beispielsweise Poly[oxy(dibutylstannylen)], Poly[oxy(dioctylstannylen)], Poly[oxy(butylphenylstannylen)] und Poly[oxy(diphenylstannylen)] (DE-OS 3 445 552), polymere Hydroxystannoxane der Formel -[-RSn(OH)-O-]-, beispielsweise Poly(ethylhydroxystannoxan), Poly(butylhydroxystannoxan), Poly(octylhydroxystannoxan), Poly(undecylhydroxystannoxan) und Poly(dodecylhydroxystannoxane) in Mengen von 0,001 bis 20 Gew.-%, bevorzugt von 0,005 bis 5 Gew.-%, bezogen auf Kohlensäurediester (DE-OS 4 006 520).

Weitere erfindungsgemäß einsetzbare Zinnverbindungen sind Sn(II)oxide und besitzen die Formel

$$X-R_2Sn-O-R_2Sn-Y,$$

worin

X und Y unabhängig voneinander OH, SCn, $OR^{11}$, $OCOR^{11}$ oder Halogen und R Alkyl, Aryl bedeuten soll (EP 0 338 760).

Als weitere erfindungsgemäß einsetzbare Katalysatoren kommen Bleiverbindungen, gegebenenfalls zusammen mit Triorganophosphanen, einer Chelarverbindung oder einem Alkalimetallhalogenid, beispielsweise $Pb(OH)_2 \cdot 2PbCO_3$, $Pb(OCO-CH_3)_2$, $Pb(OCO-CH_3)_2 \cdot 2LiCl$, $Pb(OCO-CH_3)_2 \cdot 2PPh_3$ in Mengen von 0,001 bis 1, bevorzugt von 0,005 bis 0,25 Mol pro Mol Carbonat (JP 57/176932, JP 01/093580), andere Blei(II)- und Blei(IV)-verbindungen, wie PbO, $PbO_2$, Mennige, Plumbite, und Plumbate (JP 01/093560), Eisen(III)acetat (JP 61/172852), weiterhin Kupfersalze und/oder Metallkomplexe, beispielsweise von Alkali, Zink, Titan und Eisen (JP 89/005588), Kombinationen aus Lewis-Säuren und Protonensäuren (DE-OS 3 445 553) oder Elementverbindungen von Sc, Cr, Mo, W, Mn, Au, Ga, In, Bi, Te und Lanthaniden (EP 338 760) in Frage.

Weiterhin sind in den erfindungsgemäßen Verfahren heterogene Katalysatorsysteme einsetzbar. Solche sind beispielsweise Mischoxide aus Silicium und Titan, die durch gemeinsame Hydrolyse von Silicium und Titanhalogeniden herstellbar sind (JP 54/125617) und Titandioxide mit hoher BET-Oberfläche >20 $m^2$/g (DE-OS 4 036 594)).

Bevorzugt im erfindungsgemäßen Verfahren einsetzbare Katalystoren sind Zinn-, Titan- und Zirkoniumverbindungen und die oben genannten Alkali- und Erdalkaliverbindungen, besonders bevorzugt einsetzbare Katalysatoren sind Organozinnverbindungen und Titantetraalkyl und -arylester.

Die einzusetzenden Katalysatormengen können sich teilweise von den in der Literatur genannten Mengen unterscheiden.

Beispiele 1-4:

Umesterung in einer Kombination aus einer Gegenstromumesterungskolonne A und einer Reaktionskolonnenapparatur B (siehe Fig. 4 als spezielle Ausführung von Fig. 2) mit folgendem Aufbau, wobei zusätzlich zu den oben angegebenen Bezugszeichen weitere Zeichen angegeben sind:

Gegenstromkolonne A:

Aufbau von unten nach oben beschrieben:

F1:     flüssige Sumpfentnahme über 20 cm langen Siphon am Boden der Kolonne;

G1:     Eindosierstelle für gasförmigen Dialkylcarbonatstrom aus Apparatur B am unteren Ende der Kolonne A

K1:     Isolierte Glasbodenkolonne mit 12 cm Innendurchmesser und 10 Glockenböden 12 x 10 cm (Bodenabmessung).

F2:     Einleitungsstelle für flüssigen Phenolstrom aus Kolonne B am oberen Ende der Kolonne A

G2:     gasförmige Kopfproduktentnahme über isoliertes Glasrohr zur Kolonnenapparatur B.

Reaktionskolonnenapparatur B:

Aufbau von unten nach oben beschrieben:

V1:     Fallfilmverdampfer (ca. 0,1 $m^2$ Verdampfungsfläche) mit einer füllstandsgeregelten Schwersiederproduktentnahme (P1) am unteren Ende und isoliertem Glasknie als Verbindungsstuck zur Kolonne K2;

K2:     Isolierte Bodenkolonne mit 10 cm Innendurchmesser und 5 Glockenböden 10 x 10 cm und Temperaturmeßstelle T1;

V2:     Ölthermostatisierter Intensivkühler mit einer Länge von 30 cm und einem Innendurchmesser von 7,5 cm, mit 2 innenliegenden Verdampferschlangen von je 1 cm Durchmesser (Verdampferfläche ca. 0,15 $m^2$) und Innentemperaturmesstellen am unteren und oberen Ende (T2, T3).

K3:     Isolierte Bodenkolonne mit 10 cm Innendurchmesser und 10 Glockenböden 10 x 10 cm;

F3:     Eindosierstelle für das flüssige Sumpfprodukt (F1) aus Kolonne A;

K4:     Isolierter Kolonnenschuß von 55 cm Länge und 5 cm Innendurchmesser mit SULZER-Packung DX.

B1:     Spezialschuß zur Ausschleusung des flüssigen Phenolstroms für die Kolonne A (Länge 30 cm, Innendurchmesser 5 cm) mit einem 100 ml Boden zur Phenolausschleusung (F4) zwischen zwei 50 ml Flow-Meßböden zur Messung des Flüssigkeitsstroms;

K5:     Isolierte Kolonne von 55 cm Länge und 5 cm Innendurchmesser mit SULZER-Packung DX;

E2:     Dialkylcarbonat bzw. Dialkylcarbonat/Alkohol-Eindosierstelle; (bei Anwendung von Eduktmischungen mit höherem Alkoholgehalt weiter oben, z.B. nahe E1);

B2:     Spezialboden mit (i) Gasausspeisung (G3) für die Kolonne A, (ii) einer durch ein Ventil regelbare Leitung (G4) zur regelbaren Führung eines Gasstroms in die Kolonne K6 und (iii) einem 50 cm langen variabel einstellbarem Siphon zur Rückführung des Flüssigkeitsstroms aus Kolonne K6;

K6:     Isolierte Kolonne von 55 cm Länge und 5 cm Innendurchmesser mit SULZER-Packung DX;

G5:     Gaseinleitungsrohr für Gasstrom aus Kolonne A

E1:     Phenoleindosierstelle;

K7:     Isolierter, mit einer EX-Packung der Fa. SULZER gefüllter Schuß 120 x 5 cm;

P2:     Kopfproduktentnahmestelle mit Rückflußkühler, Rücklaufteiler und Rücklaufmessung.

K = Kolonnenschüsse, V = Verdampfer/Wärmeaustauscher, B = Spezialböden, F = Flüssigein- und ausspeisestellen, G = Gasein- und ausspeisestellen, E = Edukteindosierungen, P = Endproduktentnahmestellen, T = Temperaturmeßstellen (die im Text nicht angegebenen Meßstellen können der Zeichnung entnommen werden).

Die Kopfproduktentnahmestelle (G2) der Kolonne A wird über eine isolierte Leitung mit dem Gaseinleitungsrohr (G5) der Kolonnenapparatur B verbunden. Die Sumpfproduktenahmestelle (F1) der Kolonne A wird über eine isolierte Leitung mit der Eindosierstelle (F3) der Kolonne B verbunden. Die Flüssigkeitsausspeisung (F4) am Spezialboden (B1) der Apparatur B wird über eine beheizbare Leitung mit zwischengeschalteter Dosierpumpe mit der Eindosierstelle (F2) der Kolonne A verbunden.

Die Gasausspeisung (G3) am Spezialboden (B2) der Apparatur B wird über eine isolierte Leitung mit der Gaseindosierung (G1) der Kolonne A verbunden. Das Schwersiederproduktgemisch wird am Fuß der Apparatur B über (P1), das Leichtsiederproduktgemisch über (P2) am Kopf der Apparatur B entnommen.

Die Versuche wurden in der beschriebenen Anlage nach folgender Fahrweise durchgeführt:

Anfahrprozedur und Betriebsbedingungen:

1. Die Anlage wird über E1 beim vorgegebenen Druck (in allen Beispielen Normaldruck) und auf 200°C eingestellten Verdampfern V1 und V2 unter Sollbelastung mit Phenol gefüllt, bis in der gesamten Anlage ein Phenolrückfluß in der Größenordnung der Phenoldosierung vorliegt.

2. Der Phenollauf über (F4) wird auf denselben Wert eingestellt.

3. Die Katalysator- (über 13) und DMC-Dosierung (E2) werden auf einen Sollwert fest eingestellt.

4. Bei Kopftemperaturen $T_{11}$ zwischen den in der Tabelle genannten Werten werden temperatur- und rücklaufgesteuert über P2 Gemische aus Methanol und Dimethylcarbonat entnommen.

5. Die Temperatur $T_9$ wird mit Hilfe eines aus K5 kommenden, durch das Ventil G4 geregelten Gasstroms, auf einen Sollwert eingestellt.

6. Die Phenoldosierung (E1) wird über einen Sollwert der Temperatur $T_7$ geregelt, wobei bei steigender $T_7$ die Phenoldosierung verringert, bei fallender $T_7$ erhöht wird.

7. Durch Heraufsetzen der Sumpfverdampfertemperaturen V1 und V2 werden Sollwerte der Temperaturen $T_1$, $T_2$ und $T_3$ eingestellt und am Fuß der Kolonne B über P1 Diphenylcarbonat-Produktgemische entnommen.

8. Der Phenolumlauf über (F4) wird über einen Sollwert der Temperatur $T_5$ geregelt, wobei bei steigender $T_5$ der Phenolumlauf verringert, bei fallender $T_5$ erhöht wird.

In der Tabelle sind die Versuchsbedingungen und Versuchsergebnisse zusammengestellt.

Aus den Beispielen wird deutlich, daß mit Hilfe des erfindungsgemäßen Verfahrens in einer speziellen, stofflich und energetisch gekoppelten Kolonnenkombination gleichzeitig hochangereicherte Diphenylcarbonat- und Methanolfraktionen erhalten werden können, während dies nach dem Stand der Technik nicht möglich ist. Wie aus Beispiel 4 hervorgeht, können auch Mischungen aus Dimethylcarbonat und Methanol (hier 8 Gew.-% Methanol) an geeigneter Stelle in das erfindungsgemäße Verfahren ohne Ausbeuteverlust eingesetzt werden. Die über die Sumpfverdampfer der Kolonne B eingebrachte Energie kann zwanglos zum Betreiben aller Reaktionen und Transport-, bzw. Trennvorgänge benutzt werden. Da alle Verdampfungs-und Kondensationsvorgänge direkt gekoppelt sind und an keiner Stelle extern kondensiert und wieder verdampft werden muß, ist eine optimale Ausnutzung der eingebrachten Energie möglich.

Tabelle: (Beispiele 1-4)

| Nr. | Eduktdosierung | | | Produktausspeisung | | | | | eingeregelte Temperaturen | | | | | |
| | E1 Phenol [g/h] | E2 DMC [g/h] | E3 Kataly-sator [Typ([g/h])] | [g/h] | Kopfprod. P2 Zusammen-setzung [Gew.-%] | [g/h] | Sumpfprod. P1 Zusammen-setzung [Gew.-%] | Selekt. [%] | T1 | T2 | T5 | T7 [°C] | T9 | T11 |
|---|---|---|---|---|---|---|---|---|---|---|---|---|---|---|
| 1 | 307 | 235 | **Sn-Kat. (4,3) | 193 | Methanol/53 DMC/47 | 355 | DPC/96,1 Phenol/1,8 MPC/2,3 | 99,5 | 250 | 220 | 170 | 165 | 130 | 65,5 - 64,0 |
| 2 | 280 | 225 | Ti(OPh)4 (6,2) | 185 | Methanol/50 DMC/50 | 323 | DPC/95,7 Phenol/1,3 MPC/2,9 | 99 | 250 | 220 | 170 | 165 | 130 | 65,5 - 64,0 |
| 3 | 335 | 265 | Ti(OPh)4 (14,9) | 215 | Methanol/52 DMC/48 | 384 | DPC/95,7 Phenol/1,3 MPC/2,9 | 99 | 250 | 220 | 170 | 165 | 130 | 65,5 - 64,0 |
| 4 | *Phenol 307 DMC 282 Methanol 24 | | **Sn-Kat. (4,3) | 260 | Methanol/48 DMC/52 | 352 | DPC/96,2 Phenol/1,8 MCP/2,0 | 99,5 | 250 | 220 | 175 | 167 | 120 | 65,5 - 64,0 |

* Gemisch aus Phenol, DMC und Methanol an E1 eindosiert.

** Sn-Kat. = Poly(octylhydroxystannoxan)

DMC = Dimethylcarbonat, MPC = Methylphenylcarbonat, DPC = Diphenylcarbonat

**Patentansprüche**

1. Verfahren zur Herstellung von Diarylcarbonaten der Formel

$$Ar^1\text{-}O\text{-}CO\text{-}O\text{-}Ar^1,$$

in der

$Ar^1$ unsubstituiertes Phenyl, mit 1 bis 3 $C_1$-$C_4$-Alkyl-, $C_1$-$C_4$-Alkoxy- und Halogenresten substituiertes Phenyl oder Naphthyl bedeutet,

13

durch Umesterung von aromatischen Hydroxyverbindungen der Formel

$$Ar^1OH,$$

in der

Ar$^1$ die gegebenen Bedeutungen hat,

mit 0,1 bis 10 Mol, bevorzugt mit 0,5 bis 2 Mol und besonders bevorzugt mit 0,8 bis 1,2 Mol Dialkylcarbonaten der Formel

$$R^1\text{-}O\text{-}CO\text{-}O\text{-}R^1,$$

in der

R$^1$ geradkettiges oder verzweigtes C$_1$-C$_6$-Alkyl oder C$_5$-C$_6$-Cycloalkyl bedeutet,

in Gegenwart von an sich bekannten Umesterungskatalysatoren in Kolonnenapparaturen, wie sie für Umesterungsreaktionen bekannt sind, das dadurch gekennzeichnet ist, daß die Reaktion in einer stofflich und energetisch gekoppelten Kombination aus einer Gegenstromkolonne A und einer Reaktionskolonne B, wobei in den genannten Apparaturen Reaktionen und Trennungen parallel laufen, so durchgeführt wird, daß in der Gegenstromkolonne A die aromatischen Hydroxyverbindungen, die mindestens teilweise flüssig der Reaktionskolonne B entnommen wurden, in der Flüssigphase in Gegenwart eines Umesterungskatalysators mit einem gasförmig dazu im Gegenstrom geführten Gemisch aus 100 bis 95 Gew.-Tln. Dialkylcarbonat und 0 bis 5 Gew.Tln. des zugrundeliegenden Alkohols der Formel

$$R^1OH,$$

in der

R$^1$ die gegebene Bedeutung hat,

wobei das Gemisch gasförmig der Reaktionskolonne B entnommen wurde und aromatische Hydroxyverbindungen Ar$^1$OH enthalten kann, bei Temperaturen von 100 bis 300°C und Drücken von 0,05 bis 20 bar umgesetzt wird und die in A als Sumpfprodukt anfallenden Gemische aus Alkylarylcarbonaten der Formel

$$Ar^1\text{-}O\text{-}CO\text{-}O\text{-}R^1,$$

in der

Ar$^1$ und R$^1$ die oben gegebene Bedeutung besitzen,

aus nicht umgesetzten aromatischen Hydroxyverbindungen, gegebenenfalls aus einer geringen Menge des Dialkylcarbonats und gegebenenfalls aus homogen gelöstem Katalysator in flüssiger Form in den unteren Teil der Reaktionskolonne B und die in A als Kopfprodukte anfallenden gasförmigen Gemische aus den Alkoholen, noch nicht umgesetztem Dialkylcarbonat und aromatischen Hydroxyverbindungen in den oberen Teil der Reaktionskolonne B eindosiert und bei Temperaturen von 100 bis 300°C und Drücken von 0,05 bis 5 bar im Reaktionsteil von B zu 60 bis >95 % umgesetzt werden, wobei ferner im unteren Teil der Kolonne B Diarylcarbonat als Sumpfprodukt entnommen wird, im mittleren Teil von B und oberhalb der Einspeisung des Sumpfproduktes von A der nach A zurückzuführende flüssige Strom der aromatischen Hydroxyverbindungen entnommen wird, im mittleren Teil von B zwischen der Entnahme der flüssigen aromatischen Hydroxyverbindungen und der Einspeisung des Kopfprodukts von A das nach A zurückzuführende gasförmige Gemisch aus 95 bis 100 Gew.Tln. Dialkylcarbonat und 0 bis 5 Gew.Tln. der davon abgeleiteten Alkohole und aromatischen Hydroxyverbindungen entnommen wird und als Kopfstrom von B ein Gemisch von 80 bis 20 Gew.-% der abgeleiteten Alkohole und 20 bis 80 Gew.-% Dialkylcarbonat entnommen wird, wobei umgesetztes oder entnommenes Dialkylcarbonat und umgesetzte aromatische Hydroxyverbindungen durch Einspeisung in A oder B ergänzt werden.

2. Verfahren nach Anspruch 1, dadurch gekennzeichnet, daß als aromatische Hydroxyverbindungen Phenol oder die isomeren Kresole, bevorzugt Phenol, eingesetzt werden.

3. Verfahren nach Anspruch 1, dadurch gekennzeichnet, daß Dimethyl- oder Diethylcarbonat, bevorzugt Dimethylcarbonat, eingesetzt werden.

$$R^1\text{-O-CO-O-}R^1$$

4. Verfahren nach Anspruch 1, dadurch gekennzeichnet, daß die Umsetzung in Kolonne A bei Temperaturen von 120 bis 250°C, bevorzugt bei 140-230°C durchgeführt wird.

5. Verfahren nach Anspruch 1, dadurch gekennzeichnet, daß die Umsetzung in Kolonne A bei Drücken von 0,2 bis 12 bar, bevorzugt von 0,5 bis 10 bar durchgeführt wird.

6. Verfahren nach Anspruch 1, dadurch gekennzeichnet, daß die Umsetzung in Kolonne B bei Temperaturen von 120-280°C, bevorzugt bei 140 bis 260°C durchgeführt wird.

7. Verfahren nach Anspruch 1, dadurch gekennzeichnet, daß die Umsetzung in Kolonne B bei Drücken von 0,1 bis 3 bar, bevorzugt von 0,2 bis 2 bar durchgeführt wird.

8. Verfahren nach Anspruch 1, dadurch gekennzeichnet, daß die Kolonne A eine Bodenkolonne ist.

9. Verfahren nach Anspruch 1, dadurch gekennzeichnet, daß die Kolonne B in ihrem Reaktionsteil als Bodenkolonne und in ihren Destillationstrennteilen als Füllkörper- oder Packungskolonne ausgeführt ist.

10. Verfahren nach Anspruch 1, dadurch gekennzeichnet, daß bei Einspeisung des Dialkylcarbonats in Kolonne B der zulässige Gehalt an zugrundeliegendem Alkohol im Gemisch Dialkylcarbonat/Alkohol über den Bereich von 0 bis 5 Gew.-%, bezogen auf das Gesamtgewicht des Gemisches, hinausgehen kann und 0 bis 60 Gew.-%, bevorzugt 0,5 bis 20 Gew.-%, besonders bevorzugt 1 bis 10 Gew.-% beträgt.

## Claims

1. Process for the preparation of diaryl carbonates of the formula

$$Ar^1\text{-O-CO-O-}Ar^1,$$

in which

$Ar^1$     denotes unsubstituted phenyl, phenyl substituted by 1 to 3 $C_1$-$C_4$-alkyl, $C_1$-$C_4$-alkoxy and halogen radicals, or naphthyl,

by transesterification of aromatic hydroxyl compounds of the formula

$$Ar^1\text{ - OH,}$$

in which

$Ar^1$     has the meanings given,

with 0.1 to 10 mol, preferably with 0.5 to 2 mol and particularly preferably with 0.8 to 1.2 mol, of dialkyl carbonates of the formula

$$R^1\text{-O-CO-O-}R^1,$$

in which

$R^1$     denotes straight-chain or branched $C_1$-$C_6$-alkyl or $C_5$-$C_6$- cycloalkyl,

in the presence of transesterification catalysts known per se in column apparatuses as are known for transesterification reactions, which is characterised in that the reaction is carried out in a mass-coupled and energy-coupled combination of a counter-current column A and a reaction column B, reactions and separations running in parallel in the mentioned apparatuses, in such a way that, in the counter-current column A, the aromatic hydroxyl compounds which were withdrawn at least in part in the liquid state from the reaction column B are reacted in the liquid phase in the presence of a transesterification catalyst with a mixture, conducted in counter-current thereto in the gaseous state, of 100 to 95 parts by weight of dialkyl carbonate and 0 to 5 parts by weight of the underlying alcohol of the formula

$$R^1OH,$$

in which

$R^1$    has the given meaning,

where the mixture was withdrawn in the gaseous state from the reaction column B and can contain aromatic hydroxyl compounds $Ar^1OH$, at temperatures from 100 to 300°C and pressures from 0.05 to 20 bar and the mixtures, produced in A as a bottom product, of alkyl aryl carbonates of the formula

$$Ar^1\text{-O-CO-O-}R^1,$$

in which

$Ar^1$ and $R^1$    have the meaning given above,

of unreacted aromatic hydroxyl compounds, with or without a small amount of the dialkyl carbonate and with or without homogeneously dissolved catalyst in the liquid form, are fed into the bottom part of the reaction column B and the gaseous mixtures, produced in A as head products, of the alcohols, as yet unreacted dialkyl carbonate and aromatic hydroxyl compounds are fed into the upper part of the reaction column B and are reacted at temperatures of 100 to 300°C and pressures of 0.05 to 5 bar in the reaction part of B to the extent of 60 to >95 %, where, furthermore, diaryl carbonate is withdrawn as a bottom product in the lower part of the column B, the liquid stream, which is to be returned to A, of the aromatic hydroxyl compounds is withdrawn in the central section of B and above the feed of the bottom product of A, the gaseous mixture, which is to be returned to A, of 95 to 100 parts by weight of dialkyl carbonate and 0 to 5 parts by weight of the alcohols derived therefrom and aromatic hydroxyl compounds is withdrawn in the central section of B between the draw-off of the liquid aromatic hydroxyl compounds and the infeed of the head product from A, and a mixture of 80 to 20 % by weight of the derived alcohols and 20 to 80 % by weight of dialkyl carbonate is withdrawn as a head stream from B, reacted or withdrawn dialkyl carbonate and reacted aromatic hydroxyl compounds being supplemented by feed into A or B.

2.  Process according to Claim 1, characterised in that the aromatic hydroxyl compounds used are phenol or the isomeric cresols, preferably phenol.

3.  Process according to Claim 1, characterised in that dimethyl carbonate or diethyl carbonate are used, preferably dimethyl carbonate.

$$R^1\text{-O-CO-O-}R^1$$

4.  Process according to Claim 1, characterised in that the reaction in column A is carried out at temperatures from 120 to 250°C, preferably at 140-230°C.

5.  Process according to Claim 1, characterised in that the reaction in column A is carried out at pressures from 0.2 to 12 bar, preferably from 0.5 to 10 bar.

6.  Process according to Claim 1, characterised in that the reaction in column B is carried out at temperatures from 120-280°C, preferably at 140 to 260°C.

7.  Process according to Claim 1, characterised in that the reaction in column B is carried out at pressures from 0.1 to 3 bar, preferably from 0.2 to 2 bar.

8.  Process according to Claim 1, characterised in that the column A is a tray column.

9.  Process according to Claim 1, characterised in that the column B is configured in its reaction section as a tray column and in its distillation separation sections as a dumped-packed or arranged-packed column.

**10.** Process according to Claim 1, characterised in that, when the dialkyl carbonate is fed into column B, the permissible content of underlying alcohol in the mixture of dialkyl carbonate/alcohol can pass beyond the range from 0 to 5% by weight, based on the total weight of the mixture, and is 0 to 60% by weight, preferably 0.5 to 20% by weight, particularly preferably 1 to 10% by weight.

## Revendications

**1.** Procédé pour la préparation de carbonates de diaryles de la formule

$$Ar^1\text{-O-CO-O-}Ar^1,$$

dans laquelle $Ar^1$ est un groupe phényle non substitué, un groupe phényle ou naphtyle substitué avec de 1 à 3 groupes alkyle en $C_1$-$C_4$, alcoxy en $C_1$-$C_4$ et halogènes,
par trans-estérification de composés aromatiques hydroxy de la formule

$$Ar^1OH,$$

dans laquelle $Ar^1$ a la signification donnée,
avec de 0,1 à 10 mol, de préférence avec de 0,5 à 2 mol et encore mieux avec de 0,8 à 1,2 mol de carbonates de dialkyles de la formule

$$R^1\text{-O-CO-O-}R^1,$$

dans laquelle $R^1$ est un groupe alkyle en $C_1$-$C_6$ ou cycloalkyle en $C_5$-$C_6$ linéaire ou ramifié,
en présence de catalyseurs de trans-estérification connus en eux-mêmes dans des appareils de type colonne, comme ils sont connus pour des réactions de transestérification, caractérisé en ce que l'on réalise la réaction dans une combinaison couplée en matière et en énergie d'une colonne à contre-courant A et d'une colonne de réaction B, des réactions et des séparations ayant lieu en parallèle dans les appareils cités, de telle sorte que les composés aromatiques hydroxy, qui ont été prélevés au moins partiellement sous forme liquide de la colonne de réaction B, réagissent à des températures de 100 à 300°C et à des pressions de 0,05 à 20 bar dans la colonne à contre-courant A dans la phase liquide en présence d'un catalyseur de transestérification avec un mélange gazeux introduit dans ce but à contre-courant constitué de 100 à 95 parties en poids de carbonate de dialkyle et de 0 à 5 parties en poids de l'alcool de base de la formule

$$R^1OH,$$

dans laquelle $R^1$ a la signification donnée,
le mélange ayant été prélevé sous forme gazeuse de la colonne de réaction B et pouvant contenir des composés aromatiques hydroxy $Ar^1OH$, et les mélanges obtenus dans A comme produit de queue constitués de carbonates d'alkylaryles de la formule

$$Ar^1\text{-O-CO-O-}R^1,$$

dans laquelle $Ar^1$ et $R^1$ ont la signification donnée ci-dessus,
de composés aromatiques hydroxy n'ayant pas réagi, éventuellement d'une faible quantité du carbonate de dialkyle et éventuellement de catalyseur dissous de manière homogène dans une forme liquide sont dosés dans la partie inférieure de la colonne de réaction B et les mélanges gazeux obtenus dans A comme produits de tête constitués des alcools, de carbonate de dialkyle n'ayant pas encore réagi et de composés aromatiques hydroxy dans la partie supérieure de la colonne de réaction B et sont transformés à des températures de 100 à 300°C et à des pressions de 0,05 à 5 bar dans la partie de réaction de B à de 60 à plus de 95%, du carbonate de diaryle étant prélevé comme produit de queue plus loin dans la partie inférieure de la colonne B, le courant liquide des composés aromatiques hydroxy à renvoyer dans A étant prélevé dans la partie médiane de B et au-dessus de l'introduction du produit de queue de A, le mélange gazeux à renvoyer dans A constitué de 95 à 100 parties en poids de carbonate de dialkyle et de 0 à 5 parties en poids des alcools dérivés de celui-ci et de composés aromatiques hydroxy étant prélevé dans la partie médiane de B entre le prélèvement des composés aromatiques liquides hydroxy et l'introduction du produit de tête de A et un mélange constitué de 80 à 20% en poids des alcools dérivés et de 20 à 80% en poids de carbonate de dialkyle étant prélevé comme courant de tête de B, du carbonate de dialkyle ayant réagi ou prélevé et des composés aromatiques hydroxy ayant réagi étant complétés par alimentation dans A ou dans B.

2. Procédé selon la revendication 1, caractérisé en ce que l'on utilise comme composés aromatiques hydroxy du phénol ou les crésols isomères, de préférence du phénol.

3. Procédé selon la revendication 1, caractérisé en ce que l'on utilise du carbonate de diméthyle ou de diéthyle, de préférence du carbonate de diméthyle.

$$R^1\text{-O-CO-O-}R^1$$

4. Procédé selon la revendication 1, caractérisé en ce que l'on réalise la réaction dans la colonne A à des températures de 120 à 250°C, de préférence de 140-230°C.

5. Procédé selon la revendication 1, caractérisé en ce que l'on réalise la réaction dans la colonne A à des pressions de 0,2 à 12 bar, de préférence de 0,5 à 10 bar.

6. Procédé selon la revendication 1, caractérisé en ce que l'on réalise la réaction dans la colonne B à des températures de 120 à 180°C, de préférence de 140 à 260°C.

7. Procédé selon la revendication 1, caractérisé en ce que l'on réalise la réaction dans la colonne B à des pressions de 0,1 à 3 bar, de préférence de 0,2 à 2 bar.

8. Procédé selon la revendication 1, caractérisé en ce que la colonne A est une colonne à plateaux.

9. Procédé selon la revendication 1, caractérisé en ce que la colonne B est configurée dans sa partie de réaction comme une colonne à plateaux et dans ses parties de séparation par distillation comme une colonne à corps de remplissage ou à garnissage.

10. Procédé selon la revendication 1, caractérisé en ce que pour l'introduction du carbonate de dialkyle dans la colonne B la teneur permise en alcool de base dans le mélange de carbonate de dialkyle/alcool peut dépasser le domaine de 0 à 5% en poids, rapportés au poids total du mélange, et est de 0 à 60% en poids, de préférence de 0,5 à 20% en poids, encore mieux de 1 à 10% en poids.

# Fig.1

Fig.2

# Fig.3

# Fig.4